# EUROPÄISCHE PATENTSCHRIFT

(11) **EP 4 558 062 B1**
(45) Veröffentlichungstag und Bekanntmachung des Hinweises auf die Patenterteilung: **21.10.2026**
(21) Anmeldenummer: 23751258.7
(22) Anmeldetag: 18.07.2023
(51) Int. Cl.: A61B 17/12, H01R 12/71, H01R 13/24

(54) **VERBINDUNGSKABEL UND ABLÖSEGERÄT**
CONNECTION CABLE AND DETACHMENT DEVICE
CÂBLE DE CONNEXION ET DISPOSITIF DE DÉTACHEMENT

(30) Priorität: 18.07.2022 DE 102022117911
(43) Veröffentlichungstag der Anmeldung: 28.05.2025
(73) Patentinhaber: Standard Instruments GmbH, 76189 Karlsruhe (DE)
(72) Erfinder: SCHÖNFELD, Andreas, 76189 Karlsruhe (DE); FÖLLMER, Patrik, 76185 Karlsruhe (DE)
(74) Vertreter: Schneiders & Behrendt Bochum
(86) Internationale Anmeldenummer: PCT/EP2023/069949
(87) Internationale Veröffentlichungsnummer: WO 2024/017904

(56) Entgegenhaltungen:
- EP-A1- 3 181 065
- US-B1- 6 514 280

## Beschreibung

Die Erfindung betrifft ein Kabel zur Verbindung eines elektrischen Ablösegeräts mit dem Ende eines kathetergeführten Implantats, ein Ablösegerät zur Ablösung eines kathetergeführten Implantats von einem Führungsdraht.

Kathetergeführte Implantate werden vielfach zur Behandlung vaskulärer Defekte eingesetzt. Beispielsweise werden Aneurysmen endovaskulär über einen Katheter angesteuert und mit einem kathetergeführten Implantat, beispielsweise einer Spirale aus einer Edelmetalllegierung, okkludiert. Das Implantat befindet sich an der Spitze eines Führungsdrahts und ist mit diesem über ein Ablöseelement verbunden. Ein solches Ablöseelement ist beispielsweise ein durch Elektrolyse auflösbare Metallelement oder ein thermisch abschmelzbares Kunststoffelement. Im Falle der Elektrolyse erfolgt die Ablösung des Implantats vom Führungsdraht über einen schwachen elektrischen Strom, normalerweise im Bereich von 1 mA bis 2,5 mA. Die Ablösezeit liegt im Bereich von 0,5 bis 10 s. Bei thermischen Ablöseverfahren liegt der Strom höher, typischerweise im Bereich von 100 bis 300 mA. Hierbei liegen die Ablösezeiten regelmäßig unter 1 Sekunde.

Bei der elektrolytischen Ablösung erfolgt die Zuführung des elektrischen Stroms vom Ablösegerät über den Führungsdraht zum Implantat. Der Stromkreis kann zum einen über einen innerhalb des Katheters angeordneten Draht erfolgen oder aber über eine am Patienten selbst angeordnete Rückelektrode. Im ersteren Fall ist innerhalb des Führungsdrahts und gegen diesen isoliert eine leitende Seele vorhanden, die den Rückstrom führt, im letzteren Fall erfolgt der Rückstrom über eine Nadel, die im Schulterbereich des Patienten angeordnet ist und eine Kabelverbindung zum Ablösegerät aufweist. Die Rückführung des Stroms über die Nadel geht einerseits mit einer, wenn auch geringen Verletzung einher, stellt andererseits aber auch eine möglicherweise schlechtere Verbindung für den Rückstrom her, weil unterschiedliche Körperwiderstande den Gesamtwiderstand im System erhöhen.

Da die Ströme bei der thermischen Ablösung in der Regel wesentlich höher sind, ist hier eine Stromrückführung über den Körper nicht angezeigt. Vielmehr wird hier der Heizstrom direkt im Zuführsystem über Leitungen geführt. Es sind Systeme bekannt, die einen oder zwei im Führungsdraht isoliert geführte Leitungen haben.

Zur elektrischen Verbindung der Platzierungssysteme mit oder ohne Nadel sind zwei grundsätzliche Prinzipien bekannt. Einerseits gibt es die Systeme, bei denen der Führungsdraht einen Pol darstellt, die Nadel den anderen, sogenannte monopolare Systeme, andererseits gibt es die sogenannten bipolaren Systeme, bei denen beide Pole konzentrisch angeordnet auf dem Führungsdraht liegen.

Die Implantate und die Technik zu ihrer Ablösung und Deponierung im Körper eines Patienten sind bekannt. Implantate werden beispielsweise unter der Bezeichnung Matrix, GDC und Target vertrieben. Die einzelnen Implantate haben jeweils besondere Ablösebedingungen, die von den jeweiligen Ablösegeräten eingehalten werden müssen. Solche Ablösebedingungen sind insbesondere die Stromstärke und die Ablösezeit. Die Ablösegeräte sind entsprechend an die einzelnen Implantate angepasst. Dabei berücksichtigen die Systeme auch die unterschiedliche Kontaktierbarkeit der geräteseitigen Enden der Implantatsysteme.

Die Kontaktierung der jeweiligen Anschlusspunkte erfolgt mit Klemmen oder Federkontakten, die in aller Regel über eine Goldschicht verfügen und die mechanischer Belastung unterliegen. Dies führt dazu, dass ihre Wiederverwendbarkeit begrenzt ist. Zwar kommen diese Kontaktierungen bei bestimmungsgemäßem Gebrauch nicht direkt mit dem Patienten in Kontakt, können aber dennoch z.B. mit Blut kontaminiert werden. In jedem Fall ist ihr Einsatz auf die Nutzung an einem Patienten beschränkt, da auch eine Resterilisierung mit Riskien, hohen Kosten und einer Unsicherheit über die Kontaktbeschaffenheit belastet ist.

Da der Behandlungsbereich des Patienten steril gehalten werden muss ergibt sich für Geräte, die in diesem Bereich benutzt werden ebenfalls die Forderung nach Sterilität. Dies ist insofern problematisch, als eine Resterilisierung der Geräte sehr aufwändig ist und jede Menge Restrisiken enthält, so dass gängige Praxis ist, die Geräte nach jedem Patienten zu entsorgen. Abgesehen davon, dass dies hohe Kosten verursacht, ist dies eine Verschwendung von wertvollen Ressourcen.

Dies gilt insbesondere für Geräte, wie sie in der US 2009/018 653 A1 beschrieben sind und derzeit auch eingesetzt werden. Es handelt sich um Handgeräte, die sowohl für monopolare als auch bipolare Systeme verwendbar sind. Die Geräte weisen einen Anschlussschacht auf. Da diese Geräte vom behandelnden Arzt überwacht und bedient werden, müssen sie im sterilen Bereich eingesetzt werden. Für den Zweiteinsatz bedarf es einer umfassenden Sterilisation, da eine Kontamination durch den zuvor behandelten Patienten nicht ausgeschlossen werden kann. Eine solche Sterilisation ist, sofern sie im Bereich des Anschlussschachts überhaupt möglich ist, außerordentlich aufwendig und kostenträchtig. Die Geräte werden deshalb nach einmaligem Gebrauch entsorgt.

Bei elektrolytischen Ablösesystemen hat sich etabliert, das elektronische Ablösegerät außerhalb des Sterilbereichs zu betreiben und lediglich eine Verbindungsleitung vom Gerät zum Führungsdraht des Implantats und zur Nadel zu benutzen. Diese Verbindungsleitung wird steril benutzt und wird für jeden Patienten neu benutzt. Die teure Elektronik jedoch wird wiederverwendet, da sie sich ausserhalb des Sterilbereichs befindet..

Bei bipolaren Systemen existiert so eine Lösung nicht, ebenfalls thermische Ablösesysteme sind mit bipolaren Kontakten ausgestattet und erfordern ein Gerät pro Patient.

Entsprechend ist es Aufgabe der Erfindung, ein System bereitzustellen, das es erlaubt, Ablösegeräte außerhalb des sterilen Behandlungsbereichs anzuordnen und zu betätigen, sodass eine Wiederverwendung ohne Sterilisierung möglich ist. Wünschenswert wäre ferner die Verwendung eines universellen Ablösegeräts für die Vielzahl der auf dem Markt befindlichen Implantate.

Diese Aufgabe wird mit einem Kabel zur Verbindung eines elektrischen Ablösegeräts mit dem Ende eines kathetergeführten Implantats gelöst, wobei das Kabel wenigstens zwei Strom leitende Drähte, einen Anschlussstecker zur Verbindung des Kabels mit dem Ablösegerät und einen Steckverbinder zur Verbindung mit stromleitenden Elementen des Implantats aufweist und die stromleitenden Drähte geräteseitig mit Kontakten des Anschlusssteckers verbunden sind und implantatseitig mit Kontakten des Steckverbinders und wobei das Kabel implantatseitig einen Einführtrichter aufweist, der geeignet ist, die stromleitenden Elemente des Implantats mit den Kontakten des Steckverbinders leitend zu verbinden. Ein solches Kabel ist bei entsprechender Dimensionierung für alle bipolaren Anschluss-Systeme geeignet.

Erfindungsgemäß wird unter dem Begriff geräteseitig das zum Anschluss an das Ablösegerät bestimmte Ende des Kabels verstanden und implantatseitig das zum Anschluss an das Implantat bestimmte Ende des Kabels. Unter Implantat wird im weiteren Sinne die Kombination aus dem eigentlichen Implantat am Ende des Führungsdrahts und dem Führungsdraht verstanden, wobei Führungsdraht und Implantat über das Ablöseelement miteinander verbunden sind. Im engeren Sinne ist das Implantat jeweils das zum Verbleib im Körper eines Patienten bestimmte Ende der Kombination aus Implantat und Führungsdraht. Das erfindungsgemäße Kabel ist damit zur Herstellung einer Verbindung zwischen dem Ablösegerät und dem geräteseitigen Ende des Führungsdrahts der Kombination aus Implantat und Führungsdraht bestimmt.

Das erfindungsgemäße Kabel weist wenigstens zwei stromleitende Drähte auf, die über den Anschlussstecker mit dem Ablösegerät verbunden werden können. Implantatseitig enden diese Drähte in einem Steckverbinder, der dem Anschluss an das geräteseitige Ende des Implantats - also des Führungsdrahts - dient. Die dazu erforderlichen Kontakte sind zweckmäßigerweise Kontaktfedern. Diese sind zweckmäßigerweise auf einer Platine angeordnet und werden über einen am implantatseitigen Ende des Kabels angeordneten Einführtrichter zugeführt. Eine solche Platine ist beispielsweise integral mit dem Einführtrichter verbunden und weist alle nötigen Kontakte, Leiterbahnen, etc. auf.

Zweckmäßigerweise weisen solche Kabel einen Fernauslöser auf, der es dem behandelnden Arzt ermöglicht, die Ablösung des Implantats auszulösen. Dies ist sinnvoll, da sich das Ablösegerät außerhalb des sterilen Behandlungsbereichs befindet. Eine solche Fernauslösung erfordert in der Regel mindestens einen dritten Draht innerhalb des Kabels und entsprechende Anschlusselemente auf der Platine.

Zur Erleichterung der Einführung des geräteseitigen Endes des Implantats ist es sinnvoll, den Einführtrichter mit einem auf die Dimensionen der stromleitenden Elemente des Implantats abgestimmten Durchmesser auszustatten. Es ist ferner sinnvoll, die Kontaktfedern so zu spannen, dass sie die leitenden Elemente des Implantatsystems zuverlässig kontaktieren. Gegebenenfalls kann ein Anschlag vorgesehen sein, der die Einführtiefe der stromleitenden Elemente begrenzt.

Sinnvollerweis hat der Anschlussstecker des erfindungsgemäßen Kabels Führungselemente zur Gewährleistung eines korrekten Anschlusses an das Ablösegerät sowie Rastelemente zur Herstellung einer festen Verbindung mit dem Gerät.

EP3181065 B1 offenbart ein Freisetzungssystem für eine implantierbare vaskuläre Okklusionsvorrichtung.

US6514280 B1 offenbart einen Katheter zur transvaskulären Freisetzung eines radial komprimierbaren medizinischen Geräts, der einen rohrförmigen Außenkörper aufweist, innerhalb dessen eine innere rohrförmige Hülle verschiebbar angeordnet ist, welche wiederum einen Führungsdraht aufnimmt, der axial durch die Hülle bewegbar ist.

Die Erfindung wird durch die folgenden Abbildungen bevorzugter Ausführungsformen näher erläutert. Es zeigen:
Figur 1 das implantatseitige Ende eines erfindungsgemäßen Kabels in seitlicher Ansicht;
Figur 2 das implantatseitige Ende eines erfindungsgemäßen Kabels in der Draufsicht;
Figur 3 das implantatseitige Ende des erfindungsgemäßen Kabels mit Gehäuse
Figur 4 eine Anschlussbuchse eines erfindungsgemäßen Ablösegeräts;
Figur 5 ein Kabel zur Verbindung des Ablösegeräts mit einem GDC-Implantatsystem; und
Figur 6 das Kabel von Figur vier mit integrierter Fernauslösung.

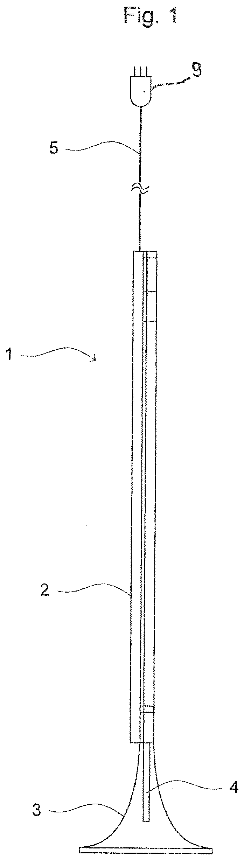

Figur 1 zeigt das implantatseitige Ende eines erfindungsgemäßen Kabels 1 in seitlicher Ansicht. Das implantatseitige Ende wird durch den Einführtrichter 3 gebildet, dass eine seitliche Verstärkung 4 aufweist. Der Einführtrichter 3 setzt an der Platine 2 an, die die zur Herstellung des Kontakts mit den leitenden Elementen des Implantats notwendigen Federzungen aufweist. Die Ziffer 5 bezeichnet das geräteseitige Ende des Kabels 1, das zum Anschlussstecker (nicht dargestellt) führt.

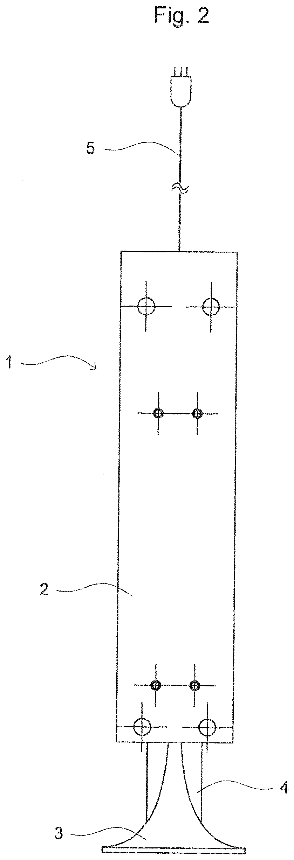

Figur 2 zeigt das implantatseitige Ende des erfindungsgemäßen Kabels 1 in der Draufsicht mit den Einführtrichter 3, den seitlichen Verstärkungen 4 sowie das geräteseitige Ende 5 des Kabels 1.

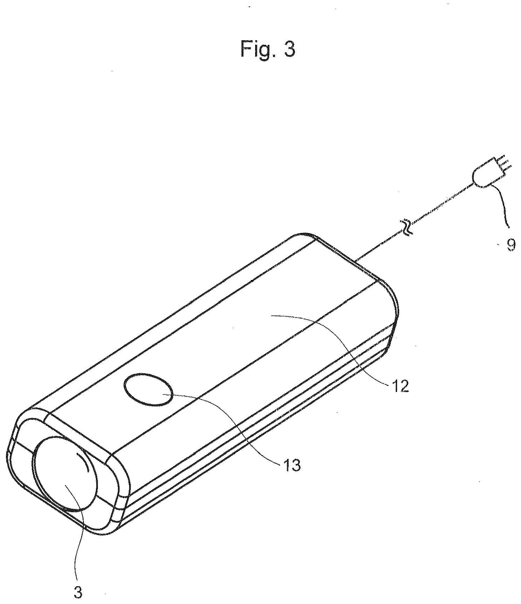

Figur 3 zeigt das implantatseitige Ende des erfindungsgemäßen Kabels 1 mit Gehäuse 12, an der Frontseite den Einführtricher 3 und den (optionalen) Fernauslösetaster 13 sowie das geräteseitige Ende mit dem Anschlussstecker 9 des Kabels 1

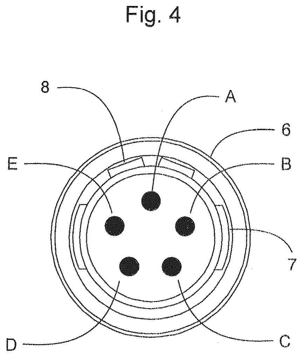

Figur 4 zeigt die Anschlussbuchse 6 des Auslösegeräts mit fünf Kontaktstiften A bis E, die beispielhaft konzentrisch im Inneren angeordnet sind, sowie zwei Rastelemente 7, die einander gegenüber liegen und zwei Führungsnasen 8, die einander benachbart zwischen den beiden Rastelemente 7 angeordnet, wobei zwischen den Führungsnasen 8 ein Zwischenraum bleibt und zu den Rastelementen 7 ebenfalls Zwischenräume. Die Zwischenräume zwischen den Führungsnasen 8 und zu den Rastelemente 7 sind unterschiedlich, was eine Erkennungsfunktion bezüglich der angeschlossenen Kabelsysteme ermöglicht. Die dargestellte Form der Anschlussbuchse ermöglicht den Anschluss zumindest dreier verschiedener Kabelsysteme mit zu den Führungsnasen und Rastelemente komplementären Elementen. Es versteht sich, dass Führungsnasen und Rastelemente konzentrisch um das Zentrum der Buchse angeordnet sind und die angeschlossenen Kabel die jeweils für das dazugehörige Ablösesystem erforderlichen Kontaktstifte kontaktieren, was den erforderlichen Ablösestrom und die erforderliche Ablösezeit auslöst. Ebenfalls möglich ist die Codierung des Steckers durch entsprechende Beschaltung der Kontakte. Werden z.B. zwei Kontakte für die eigentliche Stromführung verwendet, ein Kontakt für die Auslösung der Fernbedienung, dann bleiben in diesem Beispiel zwei weitere Kontakte für eine elektrische Codierung des Anschlusskabels. Hierbei denkbar sind alle möglichen elektrischen Signale, insbesondere. Pegel und Widerstandskombinationen.

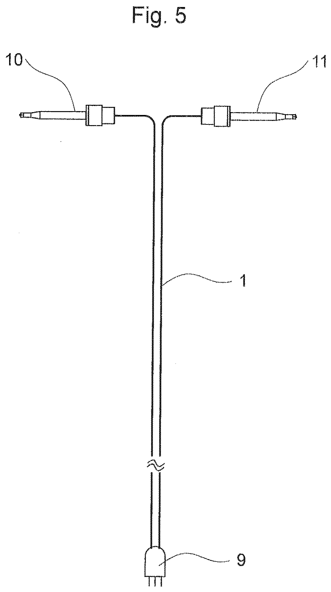

Figur 5 zeigt ein zu Figur 1 alternatives Verbindungskabel, das beispielsweise für monopolare GDC-Systeme eingesetzt werden kann. Das Kabel 1 wird über den Anschlussstecker 9 mit der Anschlussbuchse 6 des Ablösegeräts verbunden und endet in zwei Kontaktklemmen 10 und 11, die mit dem Implantat bzw. der Nadel im Schulterbereich des Patienten verbunden werden. Solche Kabel sind im Prinzip bekannt, allerdings sind sie nicht mit einer Kennung ausgestattet, die ihre Verwendung zusammen mit anderen implantatspezifischen Kabeln an ein und dem gleichen Gerät zulässt.

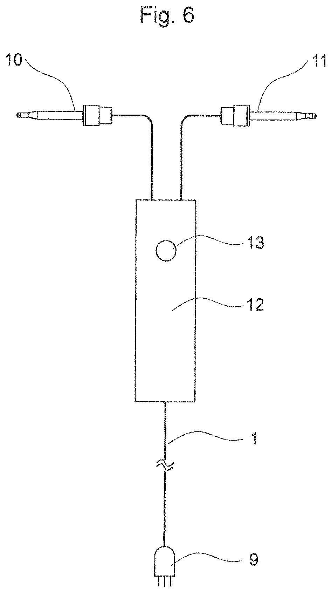

Figur 6 zeigt das Kabel von Figur 4 mit den entsprechenden Anschlüssen 9, 10 und 11 sowie einem Gehäuse 12 und zur Fernauslösung mit dem Fernauslösungsknopf 13.

## Patentansprüche

1. Kabel zur Verbindung eines elektrischen Ablösegeräts mit dem Ende eines kathetergeführten Implantats, wobei das Kabel (1) wenigstens zwei stromleitende Drähte, einen Anschlussstecker (9) zur Verbindung des Kabels (1) mit dem Ablösegerät und einen Steckverbinder zur Verbindung mit stromleitenden Elementen des Implantats aufweist und die stromleitenden Drähte geräteseitig mit Kontakten des Anschlusssteckers (9) und implantatseitig mit Kontakten des Steckverbinders verbunden sind,
**dadurch gekennzeichnet, dass**
das Kabel (1) implantatseitig einen Einführtrichter (3) aufweist, der geeignet ist, die stromleitenden Elemente des Implantats mit den Kontakten des Steckverbinders leitend zu verbinden.

2. Kabel nach Anspruch 1, **dadurch gekennzeichnet, dass** die Kontakte des Steckverbinders Kontaktfedern sind.

3. Kabel nach einem der vorstehenden Ansprüche, **dadurch gekennzeichnet, dass** die Kontakte des Steckverbinders auf einer Platine (2) angeordnet sind.

4. Kabel nach einem der vorstehenden Ansprüche, **dadurch gekennzeichnet, dass** es zwei stromleitende Drähte aufweist.

5. Kabel nach einem der Ansprüche 1 bis 3, **dadurch gekennzeichnet, dass** es drei oder mehr stromleitende Drähte aufweist, von denen mindestens einer mit einem Fernauslöser (13) verbunden ist und ggf. weitere einer elektrischen Codierung dienen.

6. Kabel nach einem der vorstehenden Ansprüche, **dadurch gekennzeichnet, dass** der Einführtrichter (3) geräteseitig einen auf die Dimensionen der stromleitenden Elemente des Implantats abgestimmten Durchmesser aufweist.

7. Kabel nach einem der vorstehenden Ansprüche, **dadurch gekennzeichnet, dass** der Anschlussstecker (9) Führungselemente (8) zur Gewährleistung eines korrekten Anschlusses an das Ablösegerät sowie Rastelemente (7) zur Herstellung einer festen Verbindung aufweist.

## Claims

1. Cable for the connection of an electrical detachment device to the end of a catheter-guided implant, with the cable (1) comprising at least two current conducting wires, a connecting plug (9) for joining the cable (1) to the detachment device, and a plug connector for connection to current conducting elements of the implant, and wherein the current conducting wires being connected on the device side to contacts of the connecting plug (9) and on the implant side to contacts of the plug connector, **characterized in that**
the cable (1) on the implant side being provided with an insertion funnel (3) which is suitable for conductively joining the current carrying elements of the implant to the contacts of the plug connector.

2. Cable according to claim 1, **characterized in that** the contacts of the plug connector are contact springs.

3. Cable according to any one of the preceding claims, **characterized in that** the contacts of the plug connector are arranged on a printed circuit board (2).

4. Cable according to any one of the preceding claims, **characterized in that** the cable is provided with at least two current carrying wires.

5. Cable according to any one of the claims 1 to 3, **characterized in that** the cable is provided with three or more current conducting wires, of which at least one is connected to a remote trigger (13) and, optionally, further wires serve for electrical coding purposes.

6. Cable according to any one of the preceding claims, **characterized in that** the insertion funnel (3) has, on the device side, a diameter which is suitably adapted to the dimensions of the current carrying elements of the implant.

7. Cable according to any one of the preceding claims, **characterized in that** the connecting plug (9) is provided with guiding elements (8) for ensuring correct connection to the detachment device as well as latching elements (7) for establishing a firm joint.

## Revendications

1. Câble destiné à relier un dispositif de détachement électrique à l'extrémité d'un implant guidé par cathéter, ledit câble (1) comportant au moins deux fils conducteurs de courant, une fiche de raccordement (9) destinée à relier le câble (1) au dispositif de détachement, et un connecteur destiné à être relié à des éléments conducteurs de courant de l'implant, et les fils conducteurs de courant étant reliés, du côté du dispositif, à des contacts de la fiche de raccordement (9) et, du côté de l'implant, à des contacts du connecteur,
**caractérisé en ce que**
le câble (1) comporte, du côté de l'implant, un entonnoir d'introduction (3) qui est apte à relier de manière conductrice les éléments conducteurs de courant de l'implant aux contacts du connecteur.

2. Câble selon la revendication 1, **caractérisé en ce que** les contacts du connecteur sont des ressorts de contact.

3. Câble selon l'une des revendications précédentes, **caractérisé en ce que** les contacts du connecteur sont disposés sur un circuit imprimé (2).

4. Câble selon l'une des revendications précédentes, **caractérisé en ce qu'**il comporte deux fils conducteurs de courant.

5. Câble selon l'une des revendications 1 à 3, **caractérisé en ce qu'**il comporte trois fils conducteurs de courant ou plus, dont au moins un est relié à un déclencheur à distance (13) et dont d'autres, le cas échéant, servent au codage électrique.

6. Câble selon l'une des revendications précédentes, **caractérisé en ce que** l'entonnoir d'introduction (3) présente, du côté du dispositif, un diamètre adapté aux dimensions des éléments conducteurs de courant de l'implant.

7. Câble selon l'une des revendications précédentes, **caractérisé en ce que** la fiche de raccordement (9) comporte des éléments de guidage (8) destinés à garantir un raccordement correct au dispositif de détachement, ainsi que des éléments d'encliquetage (7) destinés à établir une connexion solide.
